# EUROPEAN PATENT SPECIFICATION

(11) **EP 3 630 258 B1**
(45) Date of publication and mention of the grant of the patent: **20.03.2024**
(21) Application number: 18731689.8
(22) Date of filing: 25.05.2018
(51) Int. Cl.: A61M 25/09

(54) **CORE-WIRE JOINT WITH MICRO-FABRICATED MEDICAL DEVICES**
KERNDRAHTGELENK MIT MIKROHERGESTELLTEN MEDIZINISCHEN VORRICHTUNGEN
ARTICULATION DE FIL CENTRAL DOTÉE DE DISPOSITIFS MÉDICAUX MICRO-FABRIQUÉS

(30) Priority: 26.05.2017 US 201762511597 P
(43) Date of publication of application: 08.04.2020
(73) Proprietor: Scientia Vascular, Inc., West Valley City, UT 84119 (US)
(72) Inventor: DAVIS, Clark C., Holladay, Utah 84121 (US)
(74) Representative: Hepworth Browne
(86) International application number: PCT/US2018/034723
(87) International publication number: WO 2018/218191

(56) References cited:
- US-A1- 2002 049 392
- US-A1- 2003 125 641
- US-A1- 2004 254 450

## Description

### BACKGROUND

Guidewire devices are often used to lead or guide catheters or other interventional devices to a targeted anatomical location within a patient's body. Typically, guidewires are passed into and through a patient's vasculature in order to reach the target location, which may be at or near the patient's heart or neurovascular tissue, for example. Radiographic imaging can be utilized to assist the physician in navigating a guidewire to the targeted location. In many instances, a guidewire is left at a target location within the body during the interventional procedure where it can be used to guide multiple catheters or other interventional devices to the targeted anatomical location.

Tuning the flexibility of a guidewire device, particularly the distal sections of the guidewire device, is also a concern. In many circumstances, relatively high levels of flexibility are desirable in order to provide sufficient bendability of the guidewire to enable the guidewire to be angled through the tortuous bends and curves of a vasculature passageway to arrive at the targeted area. For example, directing a guidewire to portions of the neurovasculature requires passage of the guidewire through curved passages such as the carotid siphon and other tortuous paths.

Another concern related to guidewire devices is the ability of a given guidewire device to transmit torque from the proximal end to the distal end (i.e., the "torquability" of the guidewire device). As more of a guidewire is passed into and through a vasculature passageway, the amount of frictional surface contact between the guidewire and the vasculature increases, hindering easy movement of the guidewire through the vasculature passage. A guidewire with good torquability enables torqueing forces at the proximal end to be transmitted through the guidewire to the distal end so that the guidewire can rotate and overcome the frictional forces.

Some guidewire devices include a distally placed micro-machined hypotube positioned over the distal end of the guidewire core in order to direct applied torsional forces further distally toward the end of the device. Because torsional forces are primarily transmitted through the outer sections of a cross-section of a member, the tube is configured to provide a path for increased transmission of torque as compared to the amount of torque transmitted by a guidewire core not sheathed by a tube.

While such guidewire devices have provided many benefits, several limitations remain. For example, it is difficult to couple or connect superelastic materials such as nitinol to guidewire core materials and maintain desired design characteristics of the guidewire device.

US2003125641 discloses a guidewire comprising two guidewire core sections where the corresponding interface ends engage by means of complementary locking serrations.

### BRIEF SUMMARY

The present disclosure relates to guidewire devices having a joint and having effective torquability and desired bending flexibility. In the invention as defined in claim 1, a guidewire device includes a core having a proximal section, a distal section, and a joint between the proximal and distal sections. The joint includes or is formed by mechanically interlocking the distal end of the proximal section with the proximal end of the distal section. The guidewire device additionally includes a tube structure surrounding the joint.

In one embodiment, the proximal section of the core is made of or includes stainless steel and the distal section is made of or includes a superelastic material such as nitinol. Further, in the invention, the terminal, distal portion of the proximal section of the core includes serrations, and a terminal, proximal portion of the distal section of the core includes complementary serrations sized and shaped to interlock with the serrations of the proximal section. The distal end of the proximal section mechanically interlocks with a proximal end of the distal section to form the joint wherein the joint is tapered, and wherein the serrations and the complementary serrations are tapered to form the tapered joint.

In some embodiments, the joint comprises an interference fit between the serrations and the complementary serrations. Alternatively, the joint may comprise a loose fit between the serrations and the complementary serrations. In some embodiments, the joint additionally includes a medical grade adhesive. In some embodiments, the tube structure mechanically restrains the serrations and complementary serrations in an interlocked state by, for example, preventing lateral movements of the terminal, distal portion of the proximal section with respect to the terminal proximal portion of the distal section.

Additional features and advantages will be set forth in part in the description that follows, and in part will be obvious from the description, or may be learned by practice of the embodiments disclosed herein. The objects and advantages of the embodiments disclosed herein will be realized and attained by means of the elements and combinations particularly pointed out in the appended claims. It is to be understood that both the foregoing brief summary and the following detailed description are exemplary and explanatory only and are not restrictive of the embodiments disclosed herein or as claimed.

### BRIEF DESCRIPTION OF THE DRAWINGS

In order to describe the manner in which the above-recited and other advantages and features of the invention can be obtained, a more particular description of the invention briefly described above will be rendered by reference to specific embodiments thereof which are illustrated in the appended drawings. Understanding that these drawings depict only typical embodiments of the invention and are not therefore to be considered to be limiting of its scope, the invention will be described and explained with additional specificity and detail through the use of the accompanying drawings in which:
Figures 1 illustrates an example of a guidewire device not forming part of the invention having an effective joint for coupling a proximal section and a distal section of a core;
Figure 2A is a magnified, cross-sectional view of the joint and distal end of the guidewire device of Figure 1;
Figure 2B illustrates an embodiment of a guidewire device having a joint and coupling tube separate from a more distal micro-fabricated tube;
Figure 3 illustrates a cross-sectional view of the joint from the guidewire device of Figure 1;
Figure 4A illustrates a cross-sectional view of an exemplary embodiment of the present invention joint having tapered serrations;
Figure 4B illustrates a cross-sectional view of an exemplary joint not forming part of the invention having a diminishing serration; and
Figures 5 through 9 illustrate various exemplary cut patterns that may be formed in the tube structure of the guidewire device of Figure 1.

### DETAILED DESCRIPTION

### Introduction

The present disclosure relates to guidewire devices providing effective anatomical navigation capabilities. The ability to steer and direct a guidewire to a targeted anatomical location depends on balancing and optimizing tradeoffs between torquability and flexibility. Rigid materials having high torquability generally possess low flexibility, making intravascular navigation difficult. On the other hand, elastic materials that possess high flexibility often lack torquability, particularly as the distance between the site of torque application and the distal tip increases.

An advantageous guidewire device may include a proximal section having effective torquability and a distal section having effective flexibility to enable navigation of tortuous paths of the vasculature. However, it is difficult to find a single material that strikes the proper balance between torquability and flexibility, and joining two materials with different properties has also proven problematic. For example, hybrid guidewire devices comprising two materials have often required joints with long welds, joints that are difficult or incompatible for soldering, and/or joints that require long or copious amounts of adhesive to maintain association of the two materials. These are disadvantageous and sometimes counterproductive to making a guidewire with optimal torquability and flexibility. Long welds, for example, result in a large stiffness discontinuity profile, and poor soldering results in fragile guidewires.

Embodiments described herein provide one or more features that balance and/or optimize the relationship between guidewire torquability and flexibility. Such guidewires are responsive to operator manipulation during guidewire deployment, and provide effective navigation capabilities by enabling a flexible distal end to receive transmitted torsional forces.

In the invention, a hybrid guidewire device includes a proximal section and a distal section with each section comprising a different material. The proximal section and the distal section are mechanically interlocked, forming a joint. The joint is stabilized by the nature of the mechanical interlock. For example, the proximal and distal sections can include complementary and interlocking serrations that when drawn together form an interference fit. The interference fit can prevent lateral and longitudinal movements, and in some embodiments, the interference fit can prevent disassociation of the joint that could result from rotational or torsional movements. Instead, the joint is maintained and can more effectively communicate movements between the proximal and distal sections.

In the invention, the joint is stabilized by a tube structure that surrounds the joint and prevents disassociation of the proximal and distal sections by, for example, preventing lateral and longitudinal movements. As such, the interlocking serrations of the joint prevent disassociation during translational movements in the direction of the longitudinal axis, and the tube structure can prevent disassociation of the proximal and distal sections during rotational movements (e.g., axially about the longitudinal axis) and/or lateral movements (e.g., radially with respect to the longitudinal axis). In some embodiments, medical grade adhesive is applied between the proximal and distal sections as additional reinforcement to the joint. Adhesives, solder, and/or welds can additionally be used to couple the tube structure to one or more of the proximal section or the distal section.

In the present invention, the serrations are tapered . Additionally, manufacturing tolerances prevent one or more serrations from snugly interlocking with its corresponding recess, thereby leaving a space. Such as space can, in some embodiments, negligibly affect the joint. Nevertheless, in some embodiments, an adhesive can be applied within the space to further reinforce the confluence of the proximal and distal sections at the joint. It should be appreciated that other bonds-aside from adhesive-can be employed to strengthen the mechanically interlocked joint, such as, for example, bonds generated through welding or soldering.

The hybrid guidewires adding a mechanically interlocking joint that are disclosed herein provide a number of advantages that should be apparent to one having skill in the art. For example, due to the strength imparted by mechanically interlocking the proximal and distal sections, the joint can comprise a shorter segment of the core as opposed to adhesive bound joints that require a comparatively longer segment to achieve the same result. This reduces the stiffness discontinuity profile that typically blights the utility and/or operability of traditional hybrid guidewires.

Further, tapering to joint can permit a more continuous transition of material properties found in the proximal section and the distal section, and in some embodiments, the tapering can be extended over a longer distance to more slowly transition between these properties. The foregoing advantages are exemplary in nature, and it should be appreciated that additional advantages of the disclosed hybrid guidewires are evident from the figures and accompanying disclosure as well as through implementation of these devices.

### Hybrid Guidewire Devices

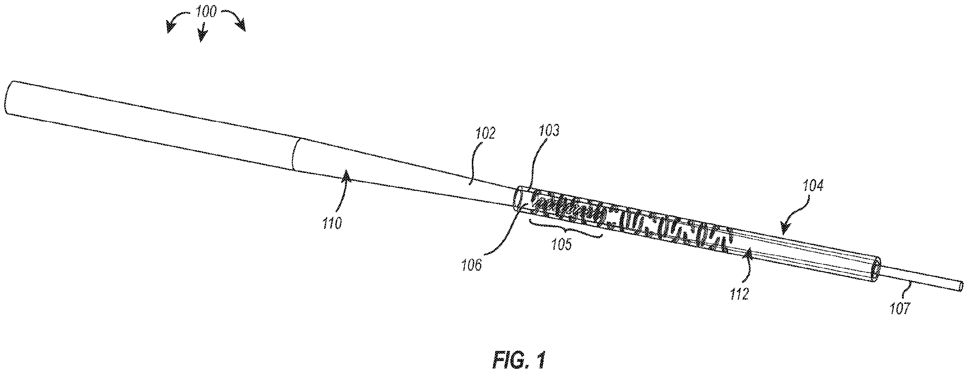

Referring now to the figures, Figure 1 illustrates an exemplary guidewire device not forming part of the invention 100 having a core 102. A tube 104 is coupled to the core 102 and extends distally from a point of attachment 103 along the core 102. As illustrated in Figure 1, the tube 104 is coupled to the proximal section 110, extends along the joint 105, and continues to the distal section 112. As shown, the distal section 112 of the core 102 and the proximal section 110 of the core 102 extend into the tube 104 and are surrounded by the tube 104. As the joint 105 comprises interlocked portions of the proximal section 110 and a distal section 112, the joint 105 is similarly encompassed by the tube 104. Figure 1 exemplifies that in some embodiments, the core 102 includes one or more tapering sections (e.g., the core 102 may be ground) so that the core 102 is able to fit within and extend into the tube 104. In the illustrated embodiment, the core 102 and the tube 104 have substantially similar outer diameters at or near the attachment point 103 where they adjoin and associate with one another.

In some examples, the proximal section 110 of the guidewire device 100 extends proximally to a length necessary to provide sufficient guidewire length for delivery to a targeted anatomical area (not shown). The proximal section 110 typically has a length ranging from about 50 to 300 cm (about 19.69 to 118.11 inches). The proximal section 110 may have a diameter of about 0.36 mm (about 0.014 inches), or a diameter within a range of about 0.20 to 3.175 mm (about 0.008 to 0.125 inches). The distal section 112 of the core 102 may taper to a diameter of about 0.051 mm (about 0.002 inches), or a diameter within a range of about 0.025 to 1.27 mm (about 0.001 to 0.050 inches). In some embodiments, the tube 104 has a length within a range of about 3 to 100 cm (about 1.18 to 39.37 inches). The tube 104 may be formed from and/or include a superelastic material such as nitinol. Alternatively, the tube 104 may be formed from and/or include a linear elastic material (e.g., with a recoverable strain of at least about 6%).

In some examples, the proximal and/or distal sections 110, 112 of the core 102 comprise round cross-sections. In other embodiments, the proximal and/or distal sections 110, 112 of the core 102 have flat or rectangular cross-sections. One or more of the proximal section 110 and/or the distal section 112 may also have another cross-sectional shape, such as a polygon shape, an arcuate shape (*e.g.*, a circle, ellipse, oval, etc.), an erratic shape, or combination of different cross-sectional shapes at different areas along its length.

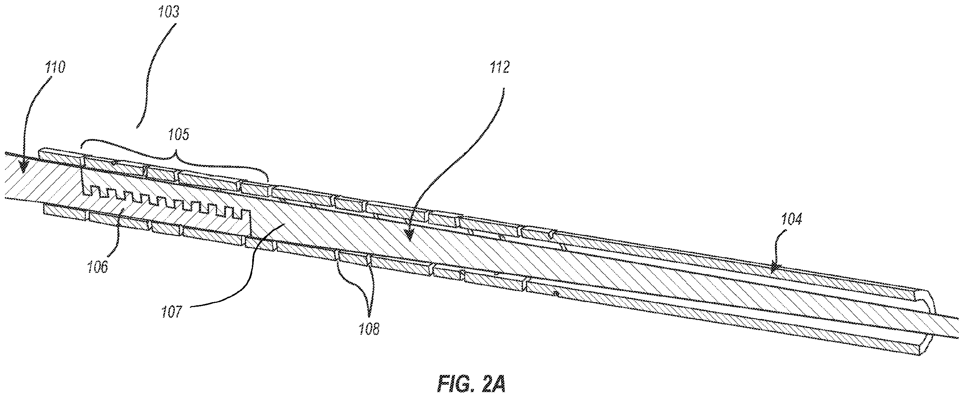

Referring now to Figure 2A, illustrated is a close-up, cross-sectional view of the joint 105 and distal section 112 of the guidewire device 100 of Figure 1. As shown, the proximal section 110 is made of and/or includes a first material 106, and the distal section 112 is made of and/or includes a second material 107. In some examples, the first material 106 is selected from materials that provide effective torquability, and the second material 107 is selected from materials that provide effective flexibility. In a preferred example, the first material 106 comprises stainless steel, and the second material 107 comprises a superelastic material such as nitinol.

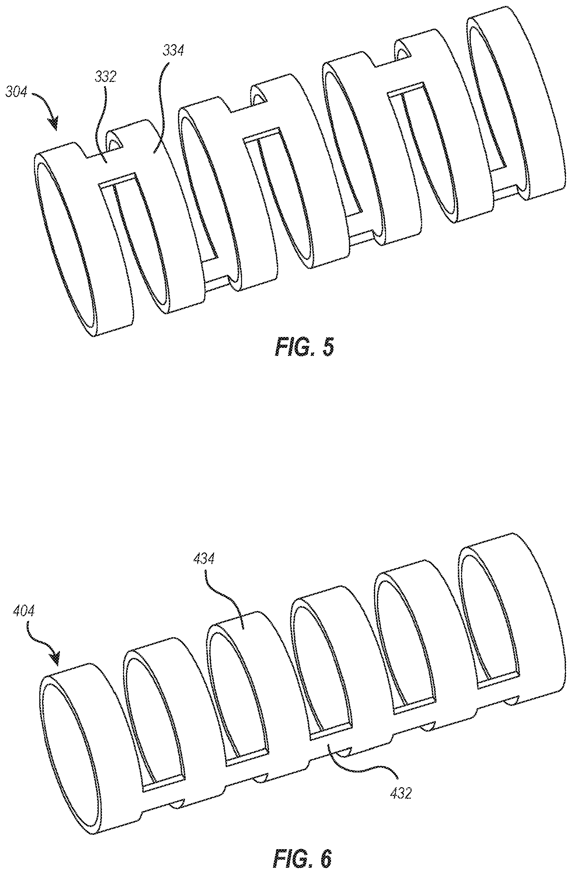

Also shown in Figure 2A, the tube 104 includes a series of cuts 108. Briefly, a series of cuts 108 can affect the flexibility (or other characteristics) of the tube 104 and can contribute to the total flex of the guidewire 100 (e.g., the combined flex of the core 102 and the tube 104), particularly where the tube 104 is disposed. In some examples, the cuts are advantageous because they allow for additional tailoring of the flexibility of the guidewire 100, including by tailoring the arrangement in which the cuts 108 are presented along the tube 104. Additionally, or alternatively, the series of cuts 108 can provide an entrance point through which adhesive may be delivered to underlying elements of the core 102 and/or for securing the tube 104 to the core 102, itself. Additional advantages, properties, and configurations of cuts 108 are provided in Figures 5 through 9 and in the corresponding text.

It should be appreciated, however, that in some examples, the tube is not cut; rather, the tube body is substantially contiguous along its length.

It will also be understood that the joint 105 may be positioned at various depths within the tube 104 (i.e., at various linear positions along the tube). For example, although the particular configuration shown in Figure 2A shows the joint 105 relatively close to the proximal end of the tube 104, the joint 105 and tube 104 may be positioned so that the joint 105 is disposed relatively deeper within the tube 104. This may be desirable in certain applications for a more secure joint 105. In some embodiments, the joint 105 is positioned at least about 1 to 5 mm within the tube 104, for example.

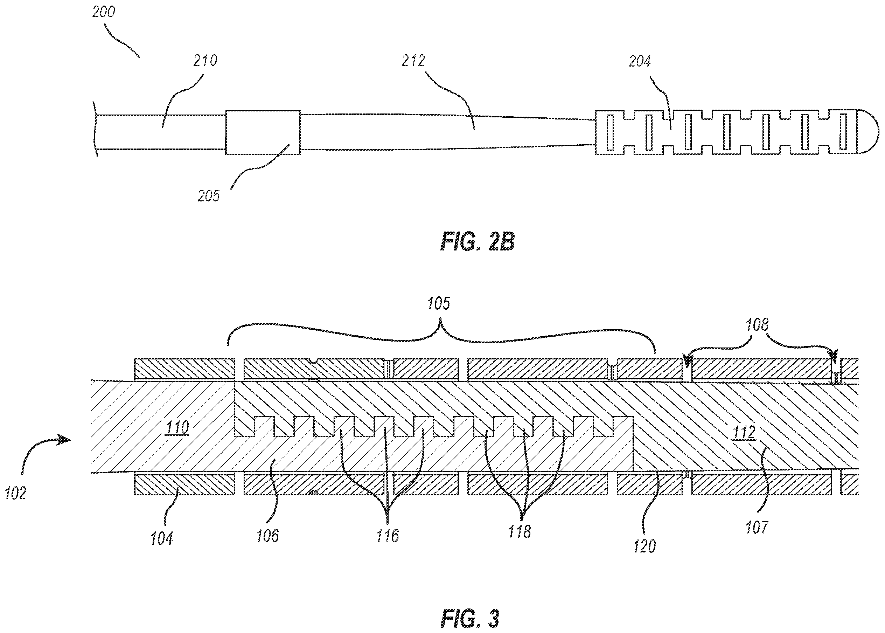

Although many of the examples provided herein show a joint positioned within a micro-fabricated tube, it will be understood that the disclosed joints may additionally or alternatively be provided at other locations along a guidewire device. For example, Figure 2B schematically illustrates a guidewire device 200 with a core having a proximal section 210 formed from a first material (e.g., stainless steel), and a distal section 212 formed from a second material (e.g., nitinol). A joint coupling the proximal section 210 and distal section 212 may be formed within the coupling tube 205. As shown, the distal section 212 may extend distally from the coupling tube 205 at full diameter (e.g., substantially equal to the diameter of the proximal section 210) before reaching a smaller or tapered diameter at the micro-fabricated tube 204.

Referring now to Figure 3, illustrated is a close-up, cross-sectional view of the joint 105. As can be seen, the joint 105 includes mechanically interlocked serrations of the proximal and distal sections 110, 112. More particularly, a terminal, distal portion of the proximal section 110 includes serrations 116 that mechanically interlock with complementary serrations 118 disposed on the terminal, proximal portion of the distal section 112 to form the joint 105. The terminal, distal portion of the proximal section 110 may be formed (e.g., by cutting) into a half round with serrations 116. Similarly, the terminal, proximal portion of the distal section may be formed (e.g., by cutting) into a half round with complementary serrations 118 that are configured in size and shape to mate with the serrations 116 of the terminal, distal portion of the proximal section 110.

The tube 104 can act to reinforce the joint 105 by mechanically constraining the joint 105. As a result, the joint 105 is prevented from disassociating due to lateral and/or rotational movements. As the joint is mechanically restrained when in combination with the tube, it should be appreciated that the joint may be formed during manufacturing by first interlocking the serrations and complementary serrations followed by sliding and/or placing the tube over the joint.

The tube 104 can be additionally secured and/or coupled to the core 102 using a medical grade adhesive 120. For example, a medical grade adhesive 120 can be used to couple the tube 104 to the proximal section 110 and/or to the distal section 112. As provided above, the medical grade adhesive 120 can be delivered to the adhesion site through one or more cuts in the tube 104. In some embodiments, the tube 104 can be coupled to the core 102 by other means, including, for example, welding, soldering, interference fit, etc. Coupling the tube 104 to the core 102 is, in some embodiments, redundant. However, doing so can, in some embodiments, ensure the integrity of the joint 105 the retaining the tube 104 in a position that the tube at least partially surrounds the joint 105.

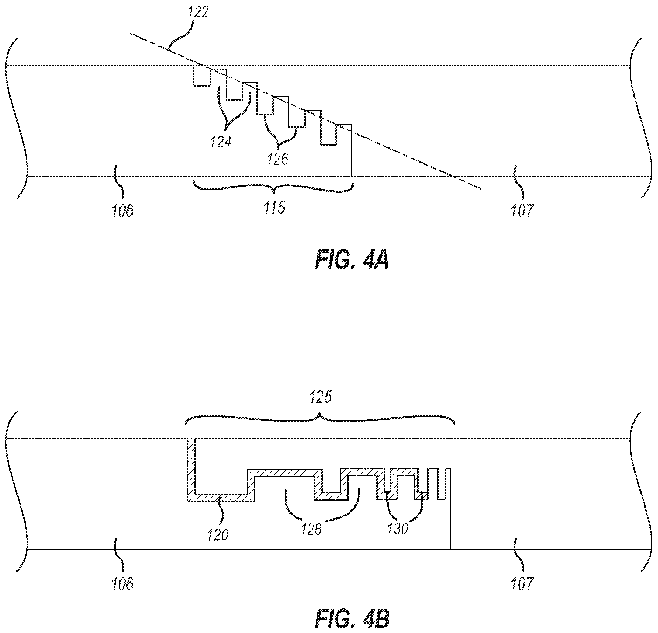

The serrations 116 and complementary serrations 118 preferably interlock as a tight fit. In some embodiments, the tight fit of the interlocking serrations and complementary serrations form an interference fit, which can be sufficient by itself to maintain structural integrity of the joint. In other embodiments, the serrations and/or the joints may take on different fits and configurations. For example, Figures 4A and 4B illustrate exemplary implementations of different serration patterns and joint configurations. Figure 4A illustrates the joint 115 of the present invention; it comprises tapered serrations 124 interlocking with complementary tapered serrations 126, and Figure 4B illustrates an exemplary joint 125 not forming part of the invention having a diminishing serrations 128 mating with complementary diminishing serrations 130.

The tube 104 can be coupled to the core 102 (e.g., using adhesive, soldering, and/or welding) in a manner that allows torsional forces to be transmitted from the core 102 to the tube 104 and thereby to be further transmitted distally by the tube 104. A medical grade adhesive 120 may be used to couple the tube 104 to the core wire 102 at the distal end of the device and to form an atraumatic covering. As explained in more detail herein, the tube 104 can be micro-fabricated to include a plurality of cuts. The cuts are arranged to form a cut pattern which beneficially provides for effective and/or directed flexibility while also maintaining good torquability.

Referring again to Figure 4A, the joint 115 is formed from interlocking tapered serrations. In some embodiments, a joint formed by interlocking tapered serrations can be stronger than other types of joints (e.g., due to the increased surface area and/or the directional retention provided thereby). Further, joints incorporating tapered serrations can introduce a continuum of bending flexibility as the joint transitions between a higher proportion of the first material 106 (with concomitant lower proportion of the second material 107) to a higher proportion of a second material 107 (with concomitant lower proportion of the first material 106) instead of a relatively more abrupt transition in materials and bending flexibility in other configurations of joints (e.g., joint 105 of Figures 1, 2A, 2B, and 3).

As shown in Figure 4A, the serrations 124 are cut along the taper line 122. The slope of the taper line 122 can influence both the length of the taper as well as the degree by which the bending flexibility transitions between the first material 106 and a second material 107. In some embodiments, the length of the taper and the degree by which the bending flexibility transitions are inversely related. Similarly, in some embodiments, the absolute value of the slope of the taper line is inversely related to the degree by which the bending flexibility transitions.

For example, as the length of the taper line (as defined between the first and last serration) increases, the degree by which the bending flexibility transitions for a given length of the core decreases. That is, a longer taper line indicates a longer joint, and consequently, there is a more finely graded transition between a first material and a second material within the joint, which results in a small transition in bending flexibility per unit length. As an additional example, as the absolute value of the slope of the taper line increases, the length of the joint decreases. A shorter joint results in a faster transition between the first material and the second material, and as a consequence of more abrupt changes in the proportions of the first and second materials per unit measure in the joint, there is a relatively higher degree of change in the bending flexibility through the joint.

As depicted in Figure 4B, the joint 125 can include diminishing serrations 128 interlocking with complementary diminishing serrations 130. As shown, the length of each individual serration diminishes compared to an adjacent serration. Figure 4B also illustrates that in some embodiments, the serrations can interlock in a loose fit. For example, the mechanical tolerances of the interlocking serrations may be liberal enough to permit non-flush fits. In such embodiments (or other embodiments disclosed herein), a medical grade adhesive 120 can be applied between the interlocking serrations to assist in forming a joint (*e.g.*, joint 125).

In general, the hybrid guidewire 100 can include one or more components formed from one or more radiopaque materials, such as platinum group, gold, silver, palladium, iridium, osmium, tantalum, tungsten, bismuth, dysprosium, gadolinium, and the like. For example, one or more radiopaque coils may be included at or near the distal tip of the device. In some embodiments, one or more coils surround the core 102 and are disposed between the core and the tube 104.

### Cut Patterns

Figures 5 through 9 illustrate exemplary examples of tube cut patterns that may be utilized in one or more of the guidewire device embodiments described herein. For example, the tube 104 of the embodiment shown in Figures 1, 2A, 2B, and 3, or a tube utilized with the components shown in Figures 4A and 4B, may be cut according to one or more of the configurations shown in Figures 5 through 9.

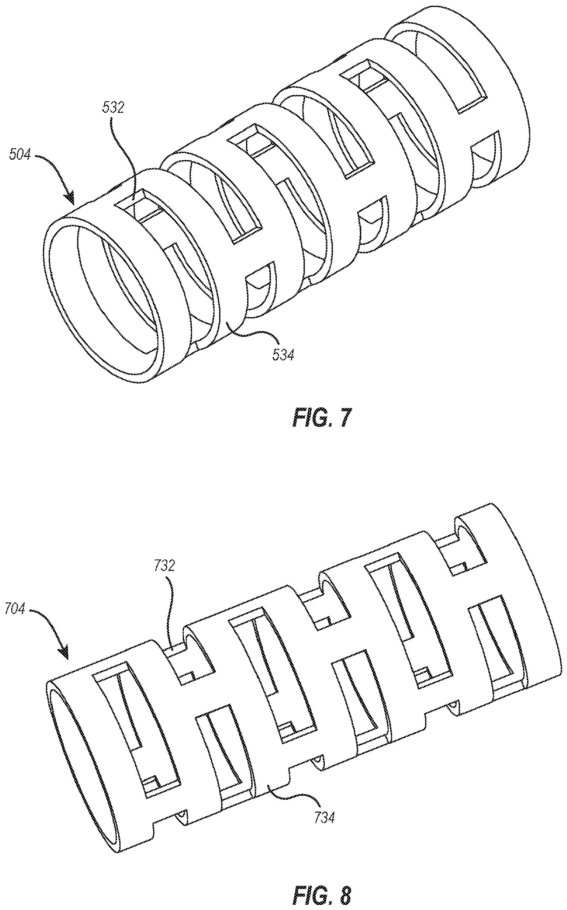

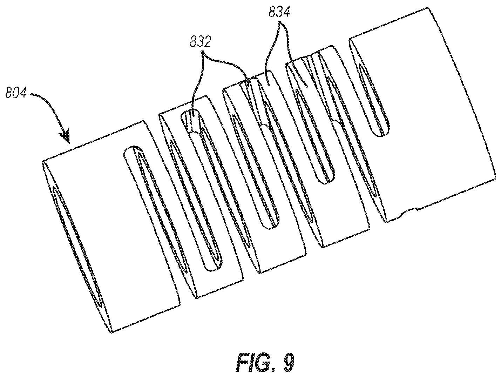

Cut patterns are referred to herein according to the number of axially extending beams disposed between each pair of adjacent circumferentially extending rings. Figures 5 and 6 illustrate "one-beam" cut patterns, Figures 7 and 9 illustrate "two-beam" cut patterns, and Figure 8 illustrates a "three-beam" cut pattern. Other examples may include more than three beams between each pair of adjacent rings (e.g., a four-beam cut pattern, five-beam cut pattern, etc.).

The tube structure 304 illustrated in Figure 5 includes a single beam 332 disposed between each pair of adjacent rings 334. Pairs of adjacent beams may alternate by 180 degrees, as shown. Additionally, or alternatively, sections may include beams positioned on a single side along a length of the tube, as shown by the beams 432 and rings 434 of the tube 404 of Figure 6.

The tube structure 504 illustrated in Figure 7 includes a pair of circumferentially opposing beams 532 disposed between each pair of adjacent rings 534. The corresponding beams 532 in each pair may be symmetrically circumferentially spaced (i.e., by about 180 degrees) as shown by Figure 7. Alternatively, the corresponding beams may be circumferentially non-symmetric. The tube structure 704 illustrated in Figure 8 includes a triad of beams 732 disposed between each pair of adjacent rings 734. The corresponding beams in each triad may be symmetrically circumferentially spaced (i.e., by about 120 degrees) as shown, or may be positioned according to some non-symmetric arrangement.

Generally, the higher the number of beams left between each pair of adjacent rings, the relatively greater the stiffness of the tube. Cut patterns may therefore be selected to provide a desired flexibility profile along the length of the tube. Cut spacing, width, and/or depth may also be varied to provide desired flexibility characteristics. For example, one tube configuration can include a proximal section with relatively lower flexibility and relatively higher torquability that rapidly progresses to a distal section with relatively higher flexibility and relatively lower torquability.

A section of tube having a two-beam cut pattern with substantially circumferentially equally spaced beams (as in Figure 7) will typically have relatively higher ability to transmit torque and relatively lower flexibility, while a section of tube having non-symmetrically spaced beams will typically have a torque transmissibility and flexibility between that of a symmetrically spaced beam pattern and a one-beam pattern. The less circumferentially symmetric the corresponding pair of beams are positioned, the closer together circumferentially the resulting beams will be, and therefore the more similar the non-symmetric two-beam cut will be to a one-beam cut pattern. Such a non-symmetric two-beam pattern may therefore be used as a transition between a symmetric two-beam pattern and a one-beam pattern.

The cut patterns may form "segments" of repeating structural units along a length of the tube. In a typical one-beam embodiment, a single segment can be defined as a first beam 332 disposed between two adjacent rings 334 (one proximal ring and one distal ring) and a second opposing beam 332 extending from the distal ring and being rotationally offset by about 180 degrees from the first beam 332. Likewise, in a typical two-beam embodiment, a single segment can be defined as a first pair of beams 532 disposed between two adjacent rings 534 (one proximal ring and one distal ring) and a second pair of beams 532 extending from the distal ring and being rotationally offset from the first pair of beams by about 90 degrees. Likewise, in a typical three-beam embodiment, a single segment can be defined as a first triad of beams 732 disposed between two adjacent rings 734 (one proximal ring and one distal ring) and a second triad of beams 732 extending from the distal ring and being rotationally offset from the first triad by about 60 degrees.

Figure 9 illustrates a tube 804 having a plurality of beams 832 and rings 834. The illustrated cut pattern includes a rotational offset applied at each successive segment of the tube 804 to minimize preferred bending directions in the tube. As used herein, a "rotational offset" is the angular rotation between two adjacent segments. A rotational offset is therefore applied from one segment to the next, even though individual cuts within a segment may also be offset from one another.

As shown, the cuts may be arranged to form a substantially consistent rotational offset from one segment to the next. The illustrated cut pattern shows a rotational offset of about 5 degrees from one segment to the next. When multiple successive segments having such an angular offset are formed, the resulting pattern of beams along a sufficient length of the tube 804 wraps around the axis of the tube 804 in a continuously rotating helical pattern. The angular offset may be about 5, 15, 30, 45, 60, 75, 80, or 85 degrees. In some examples, the angular offset is applied at each successive segment. In other embodiments, a plurality of successive segments are disposed next to one another without an offset before the angular offset is applied.

The illustrated example shows a two-beam cut pattern with a series of rotational offsets. It will be understood, however, that the same principles may be applied to other cut patterns, such as a one-beam cut pattern, three-beam cut pattern, or cut pattern having greater than three beams per pair of adjacent rings. In preferred examples, each successive cut or sets of cuts (e.g., every second cut, third, fourth, etc.) along the length of a given section is rotationally offset by about 1, 2, 3, 5, or 10 degrees, or is offset by about 1, 2, 3, 5, or 10 degrees off from 180 degrees in a one-beam pattern, 1, 2, 3, 5, or 10 degrees off from 90 degrees in a two-beam pattern, 1, 2, 3, 5, or 10 degrees off from 60 degrees in a three-beam pattern, and so on for patterns having a higher beam count. These rotational offset values have beneficially shown good ability to eliminate flexing bias.

The separate components and features of the cut patterns shown in Figures 5 through 9 may be combined to form different tube configurations. For example, some tubes may be configured so as to have a section of two-beam cuts which transitions to a section of one-beam cuts.

The examples described herein can beneficially enable more proximal regions of the tube and/or core to be tailored for transmitting torque, while tailoring more distal sections of the core and/or tube to allow for increased bending flexibility without overly sacrificing torquability. Accordingly, the features of a hybrid guidewire, including the mechanically interlocking joint and configurable tube structure, may be tuned to a particular need or application to optimize the operational relationship between torquability and bending flexibility.

The terms "approximately," "about," and "substantially" as used herein represent an amount or condition close to the stated amount or condition that still performs a desired function or achieves a desired result. For example, the terms "approximately," "about," and "substantially" may refer to an amount or condition that deviates by less than 10%, or by less than 5%, or by less than 1%, or by less than 0.1%, or by less than 0.01% from a stated amount or condition.

Elements described in relation to any embodiment depicted and/or described herein may be combinable with elements described in relation to any other embodiment depicted and/or described herein. For example, any element described in relation to a tube section of any of Figures 5 through 9 may be combined and used to form the tube 104 of the guidewire device of Figures 1 through 4B.

The present invention may be embodied in other forms, without departing from its essential characteristics. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

## Claims

1. A hybrid guidewire device (100), comprising:
a core (102) comprising:
a proximal section (110);
a distal section (112); and
a joint (105) between the proximal and distal sections, wherein the joint (105) comprises a distal end of the proximal section mechanically interlocked with a proximal end of the distal section; and
a tube structure (104) surrounding the joint,
wherein the distal end of the proximal section (110) comprises serrations (116), and wherein the proximal end of the distal section (112) comprises complementary serrations (118) configured to engage and interlock with the serrations (116) of the distal end of the proximal section (110) to form the joint (105),
wherein the joint (115) is tapered, and wherein the serrations and the complementary serrations are tapered to form the tapered joint (115).

2. The hybrid guidewire device of claim 1, wherein the core (102) comprises a circular or arcuate cross-section.

3. The hybrid guidewire device of claim 1 or claim 2, wherein the distal end of the proximal section (110) and the proximal end of the distal section (112) are truncated along a plane transverse to the longitudinal axis of the core (102) such that the joint (105) comprises overlapping the truncated distal end of the proximal section (110) with the truncated proximal end of the distal section (112).

4. The hybrid guidewire device of claim 1, wherein the joint (105) comprises an interference fit between the serrations (116) and the complementary serrations (118).

5. The hybrid guidewire device as in any one of claims 1 through 4, wherein the tube structure (104) has an interference fit with the core (102) over the joint (105).

6. The hybrid guidewire device as in any of claims 1 through 5, wherein the distal section (112) of the core (102) tapers from the proximal section (110) of the core (102) or the proximal section (110) of the core (102) tapers from the distal section (112) of the core (102).

7. The hybrid guidewire device as in any of claims 1 through 6, further comprising one or more radiopaque coils.

8. The hybrid guidewire device as in any of claims 1 through 7, wherein the core (102) is formed from stainless steel.

9. The hybrid guidewire device as in any of claims 1 through 8, wherein the tube structure is formed from a superelastic material or from a linear elastic material with a recoverable strain of at least 6%.

10. The hybrid guidewire device as in any of claims 1 through 9, wherein the tube structure (104) is formed from nitinol.

11. The hybrid guidewire device as in any of claims 1 through 10, wherein the tube structure (104) extends beyond one or more terminal ends of the joint (105) and is coupled to one or both of the proximal section (110) or the distal section (112) of the core (102).

12. The hybrid guidewire device as in any of claims 1 through 11, wherein the tube structure (104) includes a cut pattern which forms a plurality of axially extending beams coupling a plurality of circumferentially extending rings, the tube structure (104) including one or more of a one-beam cut pattern, two-beam cut pattern, or three beam-cut pattern, the cut pattern optionally including a rotational offset such that successive beams or sets of beams along a length of the tube structure (104) are circumferentially rotated with respect to a preceding beam or set of beams.

13. The hybrid guidewire device of any one of claims 1 through 12, further comprising a secondary tube structure (204) disposed distal of the tube structure (104), the secondary tube structure (204) being coupled to the distal section (112) of the core (102) and the distal section (112) of the core (102) extending within the secondary tube structure (204), the secondary tube structure (204) being micro-fabricated to include a plurality of fenestrations.

14. The hybrid guidewire device of claim 1, wherein the serrations comprise diminishing serrations (128) and the complementary serrations comprise complementary diminishing serrations (130) sized and shaped to interlock with the diminishing serrations (128).

## Patentansprüche

1. Eine Hybrid-Führungsdrahtvorrichtung (100), umfassend:
einen Kern (102), umfassend:
einen proximalen Abschnitt (110);
einen distalen Abschnitt (112); und
eine Verbindung (105) zwischen dem proximalen und dem distalen Abschnitt, wobei die Verbindung (105) ein distales Ende des proximalen Abschnitts, das mit einem proximalen Ende des distalen Abschnitts mechanisch verriegelt ist, umfasst; und
eine Röhrenstruktur (104), die die Verbindung umgibt,
wobei das distale Ende des proximalen Abschnitts (110) Verzahnungen (116) umfasst, und wobei das proximale Ende des distalen Abschnitts (112) komplementäre Verzahnungen (118) umfasst, die dazu konfiguriert sind, in die Verzahnungen (116) des distalen Endes des proximalen Abschnitts (110) einzugreifen und sich mit diesen verriegeln, um die Verbindung (105) auszubilden,
wobei die Verbindung (115) abgeschrägt ist, und wobei die Verzahnungen und komplementären Verzahnungen abgeschrägt verlaufen, um die abgeschrägte Verbindung (115) auszubilden.

2. Die Hybrid-Führungsdrahtvorrichtung nach Anspruch 1, wobei der Kern (102) einen kreisförmigen oder bogenförmigen Querschnitt umfasst.

3. Die Hybrid-Führungsdrahtvorrichtung nach Anspruch 1 oder Anspruch 2, wobei das distale Ende des proximalen Abschnitts (110) und das proximale Ende des distalen Abschnitts (112) entlang einer Ebene quer zur Längsachse des Kerns (102) gekürzt sind, so dass die Verbindung (105) eine Überlappung des gekürzten distalen Ende des proximalen Abschnitts (110) mit dem gekürzten proximalen Ende des distalen Abschnitts (112) umfasst.

4. Die Hybrid-Führungsdrahtvorrichtung nach Anspruch 1, wobei die Verbindung (105) eine Presspassung zwischen den Verzahnungen (116) und den komplementären Verzahnungen (118) umfasst.

5. Die Hybrid-Führungsdrahtvorrichtung nach einem der Ansprüche 1 bis 4, wobei die Röhrenstruktur (104) eine Presspassung mit dem Kern (102) über der Verbindung (105) aufweist.

6. Die Hybrid-Führungsdrahtvorrichtung nach einem der Ansprüche 1 bis 5, wobei sich der distale Abschnitt (112) des Kerns (102) ausgehend vom proximalen Abschnitt (110) des Kerns (102) verjüngt oder sich der proximale Abschnitt (110) des Kerns ausgehend vom distalen Abschnitt (112) des Kerns (102) verjüngt.

7. Die Hybrid-Führungsdrahtvorrichtung nach einem der Ansprüche 1 bis 6, ferner umfassend eine oder mehrere röntgendichte Spulen.

8. Die Hybrid-Führungsdrahtvorrichtung nach einem der Ansprüche 1 bis 7, wobei der Kern (102) aus Edelstahl ausgebildet ist.

9. Die Hybrid-Führungsdrahtvorrichtung nach einem der Ansprüche 1 bis 8, wobei die Röhrenstruktur aus eine superelastischen Material oder aus einem linearelastischen Material mit einer elastischen Rückstellung von mindestens 6% ausgebildet ist.

10. Die Hybrid-Führungsdrahtvorrichtung nach einem der Ansprüche 1 bis 9, wobei die Röhrenstruktur (104) aus Nitinol ausgebildet ist.

11. Die Hybrid-Führungsdrahtvorrichtung nach einem der Ansprüche 1 bis 10, wobei sich die Röhrenstruktur (104) über eine oder mehr Enden der Enden der Verbindung (105) hinaus erstreckt und mit dem proximalen Abschnitt (110) und/oder dem distalen Abschnitt (112) des Kerns (102) verbunden ist.

12. Die Hybrid-Führungsdrahtvorrichtung nach einem der Ansprüche 1 bis 11, wobei sich die Röhrenstruktur (104) ein Schnittmuster umfasst, das eine Vielzahl sich axial erstreckender Strahlen, die eine Vielzahl sich in Umfangsrichtung erstreckender Ringe verbinden, ausbildet, wobei die Röhrenstruktur (104) ein oder mehrere eines Ein-Strahl-Schnittmusters, eines Zwei-Strahl-Schnittmusters oder eines Drei-Strahl-Schnittmusters umfasst, wobei das Schnittmuster optional einen Rotationsversatz umfasst, so dass aufeinanderfolgender Strahlen oder Sätze von Strahlen entlang einer Länge der Röhrenstruktur (104) in Bezug auf einen vorhergehenden Strahl oder Satz von Strahlen in Umfangsrichtung gedreht werden.

13. Die Hybrid-Führungsdrahtvorrichtung nach einem der Ansprüche 1 bis 12, ferner umfassend eine sekundäre Röhrenstruktur (204), die distal zur Röhrenstruktur (104) angeordnet ist, wobei die sekundäre Röhrenstruktur (204) mit dem distalen Abschnitt (112) des Kerns (102) verbunden ist und sich der distale Abschnitt (112) des Kerns (102) innerhalb der sekundären Röhrenstruktur (204) erstreckt, wobei die sekundäre Röhrenstruktur (204) mikrogefertigt ist, um eine Vielzahl von Fenstern aufzuweisen.

14. Die Hybrid-Führungsdrahtvorrichtung nach Anspruch 1, wobei die Verzahnungen kleiner werdende Verzahnungen (128) umfassen und die komplementären Verzahnungen komplementäre kleiner werdende Verzahnungen (130) umfassen, die so dimensioniert und geformt sind, dass sie sich mit den kleiner werdenden Verzahnungen (128) verriegeln.

## Revendications

1. Dispositif fil-guide hybride (100), comprenant :
une âme (102) comprenant :
une partie proximale (110) ;
une partie distale (112) ; et
une articulation (105) entre la partie proximale et la partie distale, selon lequel l'articulation (105) comprend une extrémité distale de la partie proximale en verrouillage mécanique avec une extrémité proximale de la partie distale ; et
une structure tubulaire (104) entourant l'articulation,
selon lequel l'extrémité distale de la partie proximale (110) comprend des dentelures (116), et selon lequel l'extrémité proximale de la partie distale (112) comprend des dentelures complémentaires (118) configurées pour s'engager dans et se verrouiller avec les dentelures (116) de l'extrémité distale de la partie proximale (110) pour former l'articulation (105),
selon lequel l'articulation (115) est effilée, et selon lequel les dentelures et les dentelures complémentaires sont effilées de manière à former l'articulation effilée (115).

2. Dispositif fil-guide hybride selon la revendication 1, selon lequel l'âme (102) comprend une section transversale circulaire ou en forme d'arc.

3. Dispositif fil guide hybride selon la revendication 1 ou la revendication 2, selon lequel l'extrémité distale de la partie proximale (110) et l'extrémité proximale de la partie distale (112) sont tronquées le long d'un plan qui est transversal à l'axe longitudinal de l'âme (102) de sorte que l'articulation (105) comprend le chevauchement de l'extrémité distale tronquée de la partie proximale (110) avec l'extrémité proximale tronquée de la partie distale (112).

4. Dispositif fil-guide hybride selon la revendication 1, selon lequel l'articulation (105) comprend un serrage ajusté entre les dentelures (116) et les dentelures complémentaires (118).

5. Dispositif fil-guide hybride selon l'une quelconque des revendications 1 à 4, selon lequel la structure tubulaire (104) présente un serrage ajusté avec l'âme (102) par-dessus l'articulation (105).

6. Dispositif fil-guide hybride selon l'une quelconque des revendications 1 à 5, selon lequel la partie distale (112) de l'âme (102) s'effile depuis la partie proximale (110) de l'âme (102) ou la partie proximale (110) de l'âme (102) s'effile depuis la partie distale (112) de l'âme (102).

7. Dispositif fil-guide hybride selon l'une quelconque des revendications 1 à 6, comprenant en outre une ou plusieurs bobines radio-opaques.

8. Dispositif fil-guide hybride selon l'une quelconque des revendications 1 à 7, selon lequel l'âme (102) est constituée d'acier inox.

9. Dispositif fil-guide hybride selon l'une quelconque des revendications 1 à 8, selon lequel la structure tubulaire est constituée d'un matériau super-élastique ou à partir d'un matériau à élasticité linéaire présentant une déformation récupérable d'au moins 6 %.

10. Dispositif fil-guide hybride selon l'une quelconque des revendications 1 à 9, selon lequel la structure tubulaire (104) est constituée de nitinol.

11. Dispositif fil-guide hybride selon l'une quelconque des revendications 1 à 10, selon lequel la structure tubulaire (104) s'étend au-delà d'une ou plusieurs extrémités terminales de l'articulation (105) et est couplée à l'une ou aux deux parties parmi la partie proximale (110) et la partie distale (112) de l'âme (102).

12. Dispositif fil-guide hybride selon l'une quelconque des revendications 1 à 11, selon lequel la structure tubulaire (104) comporte un motif découpé formant une pluralité de traverses s'étendant axialement couplant une pluralité d'anneaux s'étendant de manière circonférentielle, la structure tubulaire (104) comportant un ou plusieurs motifs de découpe parmi un motif à une seule traverse, un motif de découpe à deux traverses, ou un motif de découpe à trois traverses, le motif de découpe comportant de manière optionnelle un décalage rotationnel de sorte que des traverses successives ou des ensembles de traverses le long de la longueur de la structure tubulaire (104) sont décalés en rotation sur la circonférence par rapport à une traverse précédente ou un ensemble de traverses précédent.

13. Dispositif fil-guide hybride selon l'une quelconque des revendications 1 à 12, comprenant en outre une structure tubulaire secondaire (204) disposée de manière distale par rapport à la structure tubulaire (104), la structure tubulaire secondaire (204) étant couplée à la partie distale (112) de l'âme (102) et la partie distale (112) de l'âme (102) s'étendant à l'intérieur de la structure tubulaire secondaire (204), la structure tubulaire secondaire (204) étant micro-fabriquée de manière à inclure une pluralité de fenêtres.

14. Dispositif fil-guide hybride selon la revendication 1, selon lequel les dentelures comprennent des dentelures décroissantes (128) et les dentelures complémentaires comprennent des dentelures décroissantes complémentaires (130) dimensionnées et formées de manière à se verrouiller avec les dentelures décroissantes (128).
